# EUROPEAN PATENT SPECIFICATION

(11) **EP 2 151 237 B1**
(45) Date of publication and mention of the grant of the patent: **09.07.2014**
(21) Application number: 08766675.6
(22) Date of filing: 19.05.2008
(51) Int. Cl.: A61K 31/197, A61K 31/5415, A61P 25/02

(54) **Pharmaceutical composition combining a non-steroidal anti-inflammatory agent and an anticonvulsant agent**
Pharmazeutische Zusammensetzung aus einem nichtsteroiden Entzündungshemmer und einem Antikonvulsivum
Composition pharmaceutique comprenant une combinaison d'un agent anti-inflammatoire non stéroïdien et d'un agent anticonvulsivant

(30) Priority: 21.05.2007 MX MX07006091
(43) Date of publication of application: 10.02.2010
(73) Proprietor: PPTM INTERNATIONAL S.à r.l., 1253 Luxembourg (LU)
(72) Inventor: GARCÍA ARMENTA, María Elena, C.P. 45020 Guadalajara, Jalisco (MX); SANTOS MURILLO, Josefina, C.P. 44600 Zapopan, Jalisco (MX); ALVAREZ OCHOA, Victor Guillermo, C.P. 45222 Zapopan, Jalisco (MX)
(74) Representative: Carvajal y Urquijo, Isabel
(86) International application number: PCT/MX2008/000060
(87) International publication number: WO 2008/143489

(56) References cited:
- EP-A1- 1 481 673
- WO-A1-99/12537
- WO-A1-99/12537
- WO-A1-2007/052999
- WO-A2-01/45683
- WO-A2-2007/093183
- US-A1- 2005 070 524
- MYUNG HA YOON: 'Evaluation of interaction between gabapentin and ibuprofen on the formalin test in rats' ANESTHESIOLOGY vol. 91, no. 4, October 1999, pages 1006 - 1013, XP008127410

## Description

The present invention has been developed to be used in the field of pharmaceutical industry and describes a pharmaceutical composition comprising the synergic combination of an non-steroidal anti-inflammatory agent, Meloxicam and an anticonvulsant agent, Gabapentin, which are formulated in a single dosage unit intended for the treatment of Neuropathic Pain caused by different etiologies.

The combination of the therapeutic agents above produces a greater effect when administered simultaneously in a single dosage unit, as opposed to when these are independently administrated, generating benefits such as reduced administration dosages, quicker therapeutic effects and fewer side effects.

### BACKGROUND OF THE INVENTION

Pain is an experience that has been part of human existence since the beginning of human creation upon the face of the earth. During the course of the different ages in which human beings have lived, they have tried, according to the extent of their knowledge, to explain the causes and mechanisms producing pain.

According to the existing culture diversity, pain has been given different meanings associated with an endless number of sensations. In our culture, pain is looked at from two main points of view: As a threat for an individual's physical and/or biological integrity, revealing a disease which cure must be found, or as the suffering caused by the deterioration of personal or work relationships, etc.

Human beings are constantly aware of numerous (internal) stimuli through their bodies, as well as (external) stimuli through the environment, reaching their cerebral cortex; these are analyzed and integrated by the brain, which emits an answer for each stimulus.

Pain is a result of different stimuli having as main function the alteration of the organism and, according to the intensity of each stimulus, it can be detrimental or not for the human body.

Pain is an unpleasant emotional (subjective) and sensorial (objective) experience associated with a tissue injury, the latter being the most frequent symptom because of which patients go to see a physician.

Pain is classified according to various categories described below: According to the Evolution Time, which can be of two types: **Chronic Pain,** which lasts more than three months and **Acute Pain,** which generally lasts about two weeks; based on their Physiology, these can be: **Physiologic Pain,** which is produced by a short stimulation of nociceptors (pain receptors) causing the activation of nociceptive pathways, manifested by a painful sensation during a few minutes and reduced tissue injury; **Inflammatory Pain,** which is stimulus that lasts longer; usually causes by tissue injury, this injury reaches an inflammatory state with permanent activation of nociceptive pathways, that can evolve to pain resolution when inflammation stops after days, or to a chronic state or neuropathic pain; and **Neuropathic Pain,** which is a nociceptive stimulus caused by a nervous injury, so that pain occurs in the presence of minimal stimuli and even in the absence of the same; and, according to the Pain Localization, it can be: **Somatic Pain,** which is a dull, continuous and localized pain, produced by the activation of nociceptors found in the skin, bones and tender parts; **Visceral Pain,** which may be a deep, oppressive and poorly localized pain, caused by the activation of nociceptors involved in processes such as: infiltration, compression, distension, traction or ischemia of pelvic, abdominal, thoracic viscera and spasms of smooth muscle in hollow viscera. When the pain is acute, it often comes with vegetative manifestations, such as nausea, vomit, sweating, tachycardia and increased arterial pressure, where pain is often present in cutaneous localizations distant from the injury provoking said manifestation.

Nowadays, Neuropathic Pain is a major problem in the field of non-neoplastic chronic pain and cancer pain, becoming a great challenge for pharmaceutical industry in terms of research and development of pharmaceutical alternatives providing benefits related to control and treatment of neuropathic pain, which is considered as one of the most common types of pain in clinical practice; however, despite of said consideration, the efficacy and success of the treatment is still distant.

Neuropathic Pain significantly influences the effective functioning and life quality of people who suffer from it, as they have difficulty to sleep, concentrate, work, walk, lack of energy, somnolence, depression and.even some people have difficulty to get dressed, due to the fact that the contact of clothes over the skin produces an unbearable burning sensation.

Neuropathic pain is caused by the presence of neurological damage of any origin in peripheral nerves, which are nervous system structures transporting the information emitted by the brain from the spinal cord to every organ in the body, and vice versa. The latter may cause motion and sensorial problems, and depending on the extent of the damage, these potential problems go from light discomfort, to paralysis or total loss of sensibility.

Neuropathic pain, product of alterations in the nociceptive transmission system, is a severe syndrome that greatly affects the patient. Diabetes mellitus is the most frequent endocrine disease and one of the main causes of neuropathic pain. Neuropathic pain is a syndrome combining a number of diseases, mechanisms, physiopathologies, topography and clinical manifestations. Neuropathic pain is not a single entity, nor a single symptom, as it is composed of a variety of positive sensorial symptoms with different physiopathological mechanisms manifested in patients in the form of pain, for these cause a negative emotion.

The same patient may suffer from more than a symptom caused by more than one different mechanism, and similarly, patients who suffer from the same symptoms respond in a different manner to the same treatment, but is not possible to predict which patients will respond to a given treatment. Chronic pain may be caused by different origins, and includes nociceptive or neuropathic pain. Nociceptive pain is a result from a mechanical, thermal or chemical stimulus, caused by direct damage or somatic/visceral tissue inflammation, which activate cutaneous or visceral nociceptors, as well as those of the musculoskeletal system, responding to the stimulus in the form of a potential action. Usually, the intensity of this kind of pain is comparable to the stimulus that triggers it, and its purpose is to protect the organism against an external aggression.

Neuropathic pain is a very complex painful syndrome with different symptoms and signs, which vary depending on time, number and intensity.

Neuropathic pain in the result from some damage or alteration in the nervous system, both at peripheral and central level, or both, producing an alteration in the nervous transmission of pain, thus, this type of pain is triggered in the absence of recognizable stimuli. It is very frequent in clinical practice, as it is observed in 2% - 40% of the population who go to medical consultation. Examples of neuropathic pain syndromes are: Clinical pictures produced by viral infections (Herpes simplex and Herpes zoster, post-herpetic neuralgia, HIV-associated neuralgia); metabolic alterations (Diabetes mellitus: diabetic neuropathy); complex regional pain syndrome; peripheral nerve trauma or compression (such as in trigerminal neuralgia or phantom limb pain); post-stroke pain (ischemic or cerebrovascular) syndrome; nerve trunk t-raumatism; some types of pain associated with neoplasia and its treatment; spinal cord alterations (such as multiple sclerosis).

Types of Peripheral Neuropathies
- Peripheral Polyneuropathy. This is the most frequent form of peripheral neuropathy. Usually, it has a symmetric and distal localization in lower extremities with a "sock-like" distribution, producing shrivelling, intense paresthesia, hyperesthesia, and pain.

The pain may be intense and burning, and it usually gets worse at night, accompanied by hiperesthesia, causing intolerance for the touch of bed sheets in patients.

Damage to the proprioceptive fibers causes alterations in gait and artropathy (Charcot's joint). During physical examination, the lack of tendinous reflexes and loss of vibratory sensibility may be observed.
- Symmetric Proximal Polyneuropathy. Mainly, this is characterized by the loss of strength, specially affecting the pelvic waist.

Diabetic Amyotrophy: An asymmetric motor deficit is produced in proximal muscles of lower extremities.

Mononeuropathies: Typically, these affect the cranial nerves (III, IV and VI). In general, these injuries tend to be unilateral and have a promising outcome.

Radiculopathy: Sensitive syndrome in which pain is present in one or more spinal nerves, mainly in the thoracic or abdominal wall.
- Autonomic Neuropathy: It may affect several organs. The gastro-intestinal apparatus is one of the most affected areas, occurring in the form of esophageal dysfunction, swallowing alterations, retarded gastric emptying, (postprandial and nocturnal) diarrhea or constipation. Occasionally, orthostatic hypotension and evident syncope. Impotence and retarded ejaculation may be present. A neurogenic bladder may require a permanent probe. Other potential manifestations are excessive sweating, dishydrosis, pupillary alterations and unnoticed hypoglycemia.

Diabetes mellitus is the most frequent endocrine disease, and its incidence is between 1% and 2% of the population. The most frequent form is non-insulin-dependent diabetes mellitus (NIDDM). Neuropathic pain is one of the most frequent complications and discomforts in diabetic patients, affecting about 45% of the diabetic population over 25 years of age and more than 50% of the diabetic patients over 60 years of age. Pain can be present in several different types of diabetic neuropathies, but its incidence and intensity degree are variable and its nature is subjective.

While it rarely causes mortality in a direct manner, it certainly causes mobility, affecting almost any part of the nervous system. Frequently, neuropathic pain in diabetic patients is associated with impacts on the mood, sleep alterations, emotional suffering, functional decline and alterations in social relationships, causing a significant decrease in the patient's life quality. However, just a minority of the patients suffering from diabetic neuropathy report clinical symptoms, thus, neuropathy can be present in the absence of symptoms. Post-herpetic neuralgia (NPH) is not a sequel to herpes zoster, but a complication thereof. Herpes zoster (HZ) is a virus-mediated disease, characterized by unilateral radicular pain, which is often accompanied by a vesicular eruption, usually limited to a dermatome innervated by the same sensitive ganglion, self-time-limited to three weeks, typically resolved without complications. NPH refers to a continuous pain across a nerve and its ramifications, generally, without inflammatory phenomena, during a period longer than one month after the cutaneous injuries have ceased to exist; other authors consider that it must be diagnosed after a two-month period.

The International Herpes Management Forum has introduced a new term, "zoster-associated pain" where pain will continue throughout the process. Hence, there may be pain during the herpes rash and acute phase and, obviously, it is necessary to determine a proper treatment, but the most relevant aspect lies in the chronic pain after the acute phase has ceased, which can last months and even years. Incidence of HZ is 125/10⁵/year in general population. Apparently, there is no seasonal, sexual or racial predisposition. The incidence of HZ is very reduced in children and adults younger than 50 years of age, increasing in geriatric population (older than 80 years of age in 5-10/10³ patients/year) probably because of a decrease in immunological terms, being the most, common cause in patients who suffer from malignant diseases and iatrogenic immunosuppression (organ transplantation, among others), and its incidence varies from 2% to 50%.

Ophthalmic HZ has an incidence of 10-17% of the HZ cases, the most frequent after thoracic HZ. It is estimated that 10% of the patients suffering from HZ will have a NPH, 50% from the population older than 60 of age and 75% of the population older than 70 years of age. According to Watson, 5% of the patients who have suffered from HZ report severe pain after a period of three months, and 2-3% after a period of one year. The most frequent clinic form is the intercostal type, affecting one or two root pairs and intercostal ganglions on one side, compared to metamerically disposed vesicles, which can gradually adopt a semicircular position, from the posterior median line to the anterior plane. Simultaneously, local pain may be experienced and its intensity may cause breathing difficulties. Bilateral HZ is rather infrequent.

The most frequent cranial nerve injury occurs in the trigerminal nerve and, usually, inflammation of Gasser's ganglion is observed Generally, the herpetic eruption is limited to a single branch, and the ophthalmic branch is the most frequently affected, causing ophthalmic HZ accompanied by pain in 93% of patients (it persists after a period of six months in 31% of the cases, a percentage that increases to 71% of cases in the population older than 80 years of age). The most frequent localizations for HZ are the following: thoracic, cranial, 1-11 trigerminal-branch and, finally, lumbar and sacral portions. Neuropathic pain not only causes an intense pain in the long term and reduces people's quality of life, but it also produces a decrease in the capacity to work and an increasing necessity of healthcare services. Nowadays, it is well known that damages to the central and peripheral nervous system produce a series of neurobiological events in the spinal cord and the cerebral structures receiving information from the damaged area. These changes are associated with sensitization, besides a great variety of clinical manifestations, including exaggerated and prolonged pain, as well as different types of evoked pain, wherein pain is extended to distant tissue structures. Neuropathic pain is representative among a heterogeneous group of diseases with different etiologies, including cancer and diabetes. Neuropathic may also differ from its localization, as disorders generated between the peripheral receptor and the brain can occur in any part of the body.

There are several examples of neuropathic pain, including: Phantom pain, due to the vast majority of long peripheral nerves; plexus avulsion pain, due to injuries in peripheral roots; and post-stroke pain, as a result of the disruption of the spinal-trigeminal-thalamic tract or its cortical projections.

Despite of the etiological heterogeneity and anatomical localization, the various types of neuropathic pain share certain characteristics, including pain in affected areas with loss of sensibility (independent stimuli), evoked pain (dependent stimuli), hyperexcitability, after-sensations, pain amount and sympathetic involvement. This combination of signs and symptoms are compatible with the sensibilization of the first, second or third order of neurons in the central nervous system, which lose part of their normal association during this intervention. Under such conditions, neuropathic pain is associated with the intervention substituted by a different and altered influence. Indeed, sensibilization is now considered a critical phenomenon behind persistent neuropathic pain. Form studies in animals and patients, new ideas concerning underlying physiopathological mechanisms and phenomena evoked by neuropathic pain have arose. The mechanisms contributing to the manifestation of neuropathic pain include:
- Pathologic activity in nociceptors or separated axons, which induce secondary changes in central neurons processing the stimuli transmitted from the spinal cord to the brain, finally producing hyperexcitability and manifestation of spontaneous and evoked pain.
- Long-term-loss of C Fibers, causing an anatomical reorganization of the dorsal horn, where long mielinated fibers grow and invade the external laminated portion of the dorsal horn, where most of the nociceptive specific neurons are localized. In this reorganization, it is assumed that there is an increased cutaneous allodynia, evoked by touch stimuli, despite of the severe nociceptor dysfunction present in allodynic skin.
- Nerve trunk inflammatory reactions, inducing the primary afferent nociceptor ectopic activity and possibly causing spontaneous pain and allodynia.

- Increased activity in the sympathetic nervous system, which can release the sympathetic terminal noradrenaline and express new receptors in afferent nociceptive neurons, resulting in an extended activity in sensitized nociceptors and persistence of pain and allodynia
- Neuroplastic changes, resulting from a peripheral or central injury, alter processing and central modulation of pain.

A useful method of differentiating neuropathic pain is through the identification of whether the pain is caused by independent or dependent (evoked) stimuli by means of standard clinical testing. There are many types of evoked pain caused by different mechanisms, which respond in different manners to a particular agent. At least in theory, this approach allows to determine the logical treatment for neuropathic pain. However, it is important to consider certain aspects. In individual patients, most of the signs and symptoms can be concomitantly present and the response to particular drugs could be compromised. Additionally, patients suffering from the same disease can have different symptoms. Finally, it is not clear yet how mechanisms transform into symptoms, and at present it is not possible to predict the success of a treatment based on symptom analysis of individual patients.

There are several molecular changes responsible for neuropathic pain, a detailed disclosure of each change is shown below. However, four of these molecular changes are worth mentioning:
1. Accumulation and new expression of peripheral calcium channels.
2. Increased activity of glutamate-receptor subpopulations, especially that of the N=methyl-D-aspartate receptor.
3. Reduction/inhibition of gamma aminobutyric (GABA-ergic) acid.
4. Changes in the penetration of calcium into cells.

Such molecular changes can be modified by certain drugs, including anticonvulsants, which constitute an important group.

The role of nitric oxide in neuropathic pain has been proven. Nitric oxide can participate in generation and perpetuation of chronic pain associated with a nervous tissue injury. Nitric oxide synthase (NOS) enzyme activity is increased in dorsal root ganglions, but decreased in spinal cords of rats with neuropathic pain caused by an injury in peripheral nerves. Also, it is known that the administration of nitric oxide synthesis inhibitors blocks the development of thermal hyperalgesia induced by a chronic constriction injury and touch allodynia induced by of ligation of spinal nerves L5/L6; wherein the latter effect is reverted by administrating the L-arginine in a dependent manner from the nitric oxide substrate doses.

Thus, data suggest that nitric oxide plays a functional role in the generation and modulation of neuropathic pain. As in other painful conditions, the role of nitric oxide' in neuropathic pain depends on the enzyme involved in its synthesis, which will determine the amount of nitric oxide generated. Apparently, the nNOS enzyme is mainly involved in neuropathic pain, as experimental data show that eNOS and iNOS enzymes are undetectable in the spinal cord and dorsal root ganglions after nerve ligation and once the manifestation neuropathic pain has fully manifested itself. However, an increase in the expression of iNOS and eNOS enzymes has been recently reported in compressed sciatic nerves, which was attributed to the enzyme expression in activated macrophages in Schwan cells, respectively, as a response to the secondary inflammation caused by nerve injury. Furthermore, the cause-and-effect relationship of nNOS enzyme expression in the generation and perpetuation of pain is also questionable.

Thus, while nerve ligation induces a mild expression of nNOS enzymes in dorsal root ganglions preceding allodynia, the expression of nNOS enzymes remains high, despite of the total resolution of allodynia and, further, it increases in animals that do not develop allodynia. Over-expression of nNOS enzymes was not observed in rats with experimental diabetes and induced neuropathy, and the treatment based on L-NAME in such rats was not effective to inhibit mechanical hyperalgesia. As a whole, these results suggest that nitric oxide produced by the over-expression of nNOS enzymes can be relevant in the degree of neuroplasticity required after a peripheral nerve injury, but do not support the nitric oxide as a direct mediator of neuropathic allodynia. As in the case of inflammatory pain, the possibility of nitric oxide having a non-inhibitory role in neuropathic pain has been proposed.

Despite of the discovery of a number of underlying mechanisms producing chronic pain, treatments presently used to control neuropathic pain have proven to be insufficient and, thus, a reconceptualization relative to the development of new pharmaceutical alternatives is necessary.

The conventional classification of neuropathic pain according to its etiology or anatomical localization has been of limited usefulness in the research of a logical treatment applicable to patients suffering from neuropathic pain.

Based on the neuronal mechanisms involved in neuropathic pain, any treatment capable of reducing its hyperexcitability is highly valuable.

Pharmacological trials carried out have focused on determining whether a treatment is effective or not through pain intensity, pain relief, patient satisfaction and drug preference assessments.

Now, pharmaceutical industry and healthcare professionals face the challenge of developing pharmacological alternatives to treat a series of potential physiopathological mechanisms, which can contribute to the manifestation of neuropathic pain. If such mechanisms are identified, an optimal treatment can be found. Following the systematic assessment of patients suffering from neuropathic pain, it should be possible to select drugs with higher effectiveness in individual patients.

### SUMMARY OF THE INVENTION

It is known that neuropathic pain, which is intense and incapacitating, tends to be resistant to the usual analgesic treatment, therefore, the main objective of the present invention is to develop a pharmaceutical composition comprised of the synergic combination of an **anticonvulsant** agent, Gabapentin, acting on specific receptors involved in the generation and perpetuation of neuronal hyperexcitability, blocking voltage-sensitive calcium channels, as well as stabilizing the membrane, and a **non-steroidal anti-inflammatory agent,** Meloxicam, acting on the serial inflammation, specially on isoenzymes COX-1 and COX-2, as well as treating all-cause moderate and severe pain, wherein this pharmaceutical composition is a safe and efficient alternative for the treatment of neuropathic pain.

### DETAILED DESCRIPTION OF THE INVENTION

The term "anticonvulsant" is a non-specific denomination for a series of components originally introduced for the treatment of epileptic convulsions, but with a variety applicable to other action mechanisms. In addition to its anti-epileptic properties, these drugs have been used to treat several chronic pain conditions, specially, neuropathic pain.

Prototype anticonvulsant agents, carbamazepine and fenitoine, were the first drugs used for the treatment of trigerminal neuralgia, based on the idea of temporal profile and on abrupt nature of painful seizures, which were similar to those observed in convulsions. Subsequently, the list has grown in order to include other painful conditions and the number of anticonvulsants has considerably increased.

Most of these drugs have shown a decrease in the ectopic discharge of injured terminal nerves and dorsal root ganglion neurons due to blockage of calcium channels. Such drugs include the following anticonvulsants: carbamazepine, oxcarbazepine, fenitoine, lamotrigine, tiagabine, topiramate and felbamate.

**Gabapentin** is a derivative from GABA neurotransmitter (gamma aminobutyric acid) and was developed as a structural analogue for the GABA system, but it does not act on GABA-ergic receptors, nor it is transformed or metabolized in GABA or GABA agonist, nor it is a GABA reuptake inhibitor. Gabapentin shows no affinity to other common receptors, such as the following: benzodiazepine, glutamate, NMDA (N-methyl-D-aspartate), α1 and α2 or β-adrenergic, collinergic, muscarinic, nicotinic, dopaminergic, histaminic, serotoninergic, opiate and canabinoid receptors, as well as voltage-dependent calcium or sodium channels. Neither has it altered dopamine, norepinephrine or serotonin reuptake.

Subsequent studies have been unable to elucidate the exact mechanism whereby Gabapentin acts; however, it is known that Gabapentin shows a high affinity and is bound to proteins called α₂δ, which are ancillary subunits of voltage-activated calcium channels present in dorsal horn post-synaptic neurons, playing a very important role in the modulation of GABA-ergic, glutaminergic and monoaminergic function, as well as in the disruption of most of the processes involved in the development of neuropathic pain.

Gabapentin is a cyclic aminoacid structurally associated with gamma aminobutyric acid (GABA), and as opposed the latter, Gabapentin is able to break through the blood brain barrier, and spread in the central nervous system in similar concentrations to those found in plasma. Gabapentin does not impede the metabolism of other anti-epileptic drugs so that there is no need to monitor or adjust dosages when administered. While being structurally related to GABA, Gabapentin action mechanism differs from other drugs involved in GABA-mediated neuronal synapsis. Gabapentin does not bind to common sites of anti-epileptic receptors or neurotransmitter receptors. Gabapentin has not been found to metabolically bind GABA or GABA agonists, and GABA reuptake or degradation has not been proven either. Studies conducted in animals with pain have shown that Gabapentin is specially efficacious for preventing neuropathic pain (spinal nerve ligation, streptozotocine-induced diabetes, Herpes zoster infection), even when it also prevents the pain caused by inflammatory processes; however, Gabapentin does not act on immediate pain.

Pharmacokinetic properties of Gabapentin predict a positive safety profile and enough bioavailability in a wide range of populations. About 50% to 60% of Gabapentin is quickly absorbed in the gastro-intestinal tract after oral administration. Maximum plasma concentrations are reached within the first 3 hours after the administration, with inter-individual and dose-dependent variability, as Gabapentin bioavailability is not proportional to the dosage: As dosages increase, bioavailability decreases. Food does not affect the rate or amount of absorbed Gabapentin. Gabapentin does not bind (or rarely bind) to plasmatic proteins (3%); it is totally cleared by the kidney in a non-metabolized form and it does not induce or inhibit hepatic enzymes. The clearance half-life of Gabapentin is from 5 to 7 hours and it is not affected by the administration of multiple doses. Specific effects over the cardiovascular or respiratory system have not been identified. While renal failure reduces the clearance of Gabapentin in adults, age is not a decisive factor for the elimination. Significant interactions between Gabapentin and other conventional anti-epileptic agents or oral contraceptives have not been observed. Gabapentin is widely distributed within the body. Maximum plasma concentrations are reached within the first 3 hours after the administration.

Non-steroidal anti-inflammatory drugs (NSAIDs) have been independently used for the treatment of neuropathic pain with less optimal results, given the fact that NSAIDs have a cyclooxigenase(COX)-specific activity, and actively participate in the reduction of inflammatory processes.

The potential benefits of NSAIDs are clinically assessed against the potential risks caused by its use, which can be significant, especially in terms of gastro-intestinal toxicity, potential development of renal failure and precipitation of congestive heart failure in susceptible people.

Meloxicam is characterized by being a strong anti-inflammatory agent used in several conventional inflammatory models; further, it has shown a minimal gastric ulcerogenicity in rat stomachs, despite of its strong anti-inflammatory activity. When the results from the first clinical studies carried out with Meloxicam were obtained, there was no explanation relative to the best pharmacological profile of Meloxicam when compared to conventional NSAIDs. Back then, only one cyclooxigenase (COX) was known, the enzyme responsible for prostaglandin synthesis, and it was thought that COX-activity inhibition caused both therapeutic effects and adverse effects of NSAIDs.

COX inhibition and, thereby, prevention of prostaglandin formation, provided a unifying explanation for the therapeutic action, gastrotoxicity, nephrotoxicity and anti-thrombotic effects of NSAIDs. Based on the discovery of a second COX enzyme, COX-2, and a hypothesis stating that the anti-inflammatory effects of NSAIDs are obtained through a mechanism different from the frequently observed adverse effects of these compounds, including alterations of the cytoprotection in the stomach, renal function and platelet-aggregation inhibition has been proposed. COX-1 is the constitutive enzyme found under physiological conditions in most tissues, it is a "housekeeping" enzyme; whereas the expression of the same isoenzyme COX-2 is mostly induced, especially during inflammatory processes. However, there is recent evidence indicating that the expression of the isoenzyme COX-2 is also constitutive in certain tissues, such as the Central Nervous System and the kidney.

Presently, most NSAIDs are used to inhibit both enzymes in a non-selective manner, causing the manifestation of anti-inflammatory effects (relative to COX-2 inhibition), but also the emergence of adverse effects (relative to COX-1 inhibition), generally of gastro-intestinal nature. Since then, it has been proven that Meloxicam is a stronger COX-2 inhibitor than it is for the isoenzyme COX-1, in recommended anti-inflammatory doses.

**Meloxicam** (4-hydroxy-2-methyl-N-(5-methyl-2-thiazolyl)-2H benzotiazine-3-carboxamide-1,1-dioxide) is a non-steroidal anti-inflammatory drug with anti-inflammatory, analgesic and anti-pyretic functions. It is a derivative from enolcarboxamide contained in the enolic acid group (pertaining to oxicams: pyroxicam, tenoxicam, sudoxicam). These have a strong ciclooxigenase-2 (COX-2)-inhibiting activity with a 75-fold selectivity for COX-2 when compared to COX-1, acting as a prostaglandin synthesis inhibitor, said prostaglandins serve as mediators responsible for the inflammatory processes.

The selective and specific blockage carried out on cyclooxigenase-2 provides Meloxicam with a double therapeutic benefit by obtaining, on one hand, a remarkable anti-inflammatory and analgesic activity, and on the other hand, it is exceptionally tolerated with minimum gastro-intestinal or ulcerogenic adverse effects, as opposed to the patients treated with other non-steroidal anti-inflammatory agents, such as indometacin, diclofenac, piroxicam, naproxen or acetylsalicylic acid.

The 5-methyl group in the thiazolyl ring of Meloxicam can enter an additional space in the COX-2 active site, explaining part of its selectivity. The effective intracellular access of Meloxicam is determined by its lipophilic and amphiphilic properties. Meloxicam low hydrosolubility with acid pH and its amphiphilic protonation behaviour are responsible for tissue kinetics, which prevent increases in the concentrations of this active principle in certain tissues of the alimentary canal.

In its acid form, Meloxicam has a membrane solubility 10-fold higher than that of Piroxicam and it comes out from membranes about 2-fold quicker than Diclofenac.

In general, Meloxicam is rapidly carried through membranes, both within a range allowing it to interact efficiently with its target enzyme (COX-2); accordingly, it does not show the typical "ionic entrapment" characteristic of most carbonic-acid-class NSAIDs, contributing to a clinically observed favourable profile of gastro-intestinal tolerability.

Meloxicam has a good digestive absorption and optimal bioavailability (89%), after the administration of a single oral dose. Meloxicam is virtually insoluble in water. Approximately 50 minutes after the administration, a concentration of 1.6 µg/mL is obtained.

Maximum plasma concentrations are obtained 6 to 9 hours after the oral administration. Some of the main pharmacokinetic characteristics are the following: Prolonged absorption, sustained serum concentrations and long elimination half-life (20 hours), allowing for the administration of a single daily dose. Meloxicam is mainly metabolized by oxidation of the thiazolyl molecule in the methyl group. Once absorption has been completed in the alimentary canal, Meloxicam is easily spread towards blood and swollen tissues, having high adherence to plasmatic proteins (99%). Around a half of the administered dose is eliminated in urine, whereas the rest is excreted in faeces.

The pharmaceutical composition object of the present invention is comprised of the pharmaceutically acceptable combination of an anticonvulsant agent, Gabapentin, and a non-steroidal anti-inflammatory agent, Meloxicam, besides pharmaceutically acceptable excipients formulated in a single dosage unit intended for oral administration, which produce an efficacious synergic effect, with improved tolerance and less manifestation of adverse effects, using reduced concentrations of the above agents, when compared to doses commonly used for the individual administration of said active principles.

In order to assess the efficiency and tolerance of the pharmaceutical composition object of the present invention, as well as the synergic effect of the active principles, Gabapentin and Meloxicam, combined in a single dosage unit; a comparative study was conducted, wherein the above active principles were individually administered; as well as the combination thereof.

By means of the study described below, the oral administration of the active principle Gabapentin, in combination with the active principle Meloxicam, there is a more efficacious effect and a stronger therapeutic activity in terms of pain relief, in an experimental animal model, wherein peripheral inflammatory nociception was induced by the intraplantar injection of A-carrageenin in a rat paw. In this study, an isobolographic analysis was conducted in order to test the interaction between Gabapentin and Meloxicam administered under three different conditions.

### Materials and Methods:

Sprague-Dawley male rats were used, with an approximate weight of 200-300 g. The animals were kept in cages with 12-hours-light/dark cycles, and water and food were provided ad lib. Rats were used once and they only received one dose of the drugs or the combination thereof.

Rats were placed in a spin cylinder to 4 rpm and one of their paws was connected to a computer terminal. After acclimatization for 1 hour, rats were administered 100 µl of 1% A-carrageenin solution on the plantar surface of one rear paw. Baseline was determined after 2.5 hours to confirm the presence of hyperalgesia. The administered vehicle in a volume of 2 mg/kg by body weight was Gabapentin (3.0-300.0 mg/kg), Meloxicam (0.1-30.0 mg/kg), or the mixture of Gabapentin and Meloxicam (0.0001-300.0 mg/kg of total dose).

Response latencies for the ipsilateral and contralateral paw were determined 30, 60, 90 and 120 minutes after the administration of the drug.

Gabapentin and Meloxicam were administered in fixed dosage ratios of 50:1, 10:1, 1:1. These fixed dosage ratios were selected by a DE50 value range for each drug and reciprocal ratio. Drug administration time was based on early findings of A-carrageenin-induced hyperalgesia, which maximum value was 2.5 hours and it remained stable for at least 2 additional hours.

Paw volume was determined by pletismometry before the injection of A-carrageenin and 2.5 hours after the injection of A-carrageenin (just before the injection of the drug 2 hours after the administration of the same).

After such assessments, rats were anesthetized with halothane and blood samples were taken for chromatographic testing.

Two analyses of variance were conducted for repeated assessments in order to compare the effects of Gabapentin, Meloxicam and the combination of Gabapentin/Meloxicam, as well as the control (vehicle). The dosage-response relationship of Gabapentin, Meloxicam and the combination of Gabapentin/Meloxicam was determined using the SLP (Paw Latency Suspension) obtained 120 minutes after the administration of the drug at the same time as the peak effect.

In order to determine the interaction nature, the dosage-derived relationship-response for the total dosage of Gabapentin and Meloxicam, was compared to the theoretical dose relationship-additive by a standard parallel line method.

### Results:

### Effects of independently administered Gabapentin and Meloxicam.

The administration of Gabapentin 3.0 - 300.0 mg/kg after inflammation induction produced a high dependent time and dosage in the ipsilateral paw SLP. The peak effect of Gabapentin occurred after 120 minutes. Contralateral paw response latencies did not show any alteration. The DE50 value for Gabapentin was 19.2 mg/kg (5.4-43.1 mg/kg range).

The administration of Meloxicam 0.1 - 30 mg/kg after inflammation induction produced a high dependent time and dosage in the ipsilateral paw SLP but not in the non-swollen contralateral rear paw. The peak effect of Meloxicam occurred after 120 minutes. The DE50 value for Meloxicam was 0.46 mg/kg (0.04 - 1.37 mg/kg range).

The dosage-response relationship was determined 2 hours after the administration of Gabapentin or Meloxicam for the rear ipsilateral paw.

### Effects of concomitantly administered Gabapentin and Meloxicam.

The relationship dosage-response for each' fixed dosage of the combination comprised of Gabapentin/Meloxicam was determined 2 hours after of the drug administration, as well as the corresponding additive theoretical dose-response.

Each combination produced a significant improvement of the A-carrageenin-induced hyperalgesia. The time courses for the anti-hyperalgesic effect were similar for each fixed dose ratio and their maximum effects occurred within 120 minutes after the oral administration. Neither combination of Gabapentin and Meloxicam had an anti-nociceptive effect on the non-injured contralateral paw.

For the fixed dose ratio of Gabapentin/Meloxicam, 50:1, (about the ratio value resulting from the DE50 values obtained by the independent administration of the active principles), the total dose of 10.0 mg/kg significantly increased the swollen rear paw SLP. The dose-response relationship for the combination was placed to the left of the theoretical additive dose line and was significantly different from the additive dose line, consisting in a synergic interaction.

For the fixed dose ratio of Gabapentin/Meloxicam, 10:1, total dose 1.0 mg/kg significantly increased the rear swollen paw SLP. The dose-response relationship for the combination was placed to the left of the theoretical additive dose line and was significantly different from the additive dose line, consisting in a synergic interaction.

For the fixed dose ratio of Gabapentin/Meloxicam, 1:1, total dose of 0.01 mg/kg or more significantly increased the rear swollen paw SLP. The dose-response relationship for very low doses of the combination was to the left of the theoretical additive dose line and was significantly different from the additive dose line, consisting in a synergic interaction.

The administration of a total dose of 10 mg/kg with a dose ratio of 1:1 did not increased the SLP by more than 0.1 mg/kg of the total dose, which was sufficient to revert the hyperalgesia.

Isobolographic analyses using ratios of 50:1, 10:1 and 1:1 (Gabapentin/Meloxicam) showed a synergic interaction between both active principles, whereas the analysis using a ratio of 1:50 (Gabapentin/Meloxicam) only showed an additive interaction.

Chromatographic analyses of samples suggest that the additional presence of Meloxicam does not alter plasma concentrations of Gabapentin.

The mechanisms responsible for the synergic interaction between Gabapentin/Meloxicam are due to the pharmacokinetics of each active principle, as it has been shown that none of these active principle levels is altered when combined.

### Mechanisms through which Gabapentin and Meloxicam can interact.

Apparently, the sites where Gabapentin produces an anti-hyperalgesic effect (mainly in the spinal cord) and sites where Meloxicam acts (spinal cord and peripheral area) are complementary. Pathways through which these active principles act are different. This profile can be the base for a synergic interaction between components. The sensitizing and the correlation of peripheral inflammation-induced behaviour is partly mediated by action of glutamate found in NMDA receptors, which produce a calcium influx, neuronal excitability increased and eventual prostaglandin up-regulation. Gabapentin reduces the calcium influx though a number of mechanisms, Meloxicam is able to synergize these effects by means of PGE2-synthesis inhibition, which may cause a minor glutamate release in presynaptic neurons and, thus, reduce dorsal horn neuronal excitability.

Synergetic interactions of Gabapentin/Meloxicam are reflected as an increase the power and efficacy of the combination. The main advantage of this combination is the ability to administrate reduced doses of the active principles comprised therein, which produce a significant reduction in hyperalgesia when combined. This constitutes a therapeutic advantage in terms of chronic neuropathic pain, especially among geriatric patients.

## Claims

1. A pharmaceutical composition **characterized in that** comprises Gabapentin and Meloxicam, as well as pharmaceutically acceptable excipients; wherein concentration ranges for Gabapentin are from 3.0 mg to 300.0 mg and from 0.1 mg to 30.0 mg for Meloxicam; these are formulated in a single dosage unit for oral administration.

2. The pharmaceutical composition of claim 1, **characterized in that** Gabapentin is present in the formulation in a concentration of 300.0 mg per dosage unit.

3. The pharmaceutical composition of claim 1 and 2, **characterized in that** Meloxicam is present in a concentration of 7.5 mg per dosage unit.

4. The pharmaceutical composition of claims 1 to 3, **characterized in that** such composition is formulated in a single dosage unit in the form of capsules or tablets.

5. The pharmaceutical composition of claims 1 to 4, **characterized in that** such composition is for use in the treatment of Neuropathic Pain.

## Patentansprüche

1. Pharmazeutische Zusammensetzung, **dadurch gekennzeichnet, dass** sie Gabapentin und Meloxicam, sowie pharmazeutisch verträgliche Hilfsstoffe umfasst; wobei die Konzentration von Gabapentin 3,0 mg bis 300,0 mg und die Konzentration von Meloxicam 0,1 mg bis 30,0 mg beträgt; wobei diese sind in einer Einzeldosiseinheit zur oralen Verabreichung formuliert.

2. Pharmazeutische Zusammensetzung nach Anspruch 1, **dadurch gekennzeichnet, dass** Gabapentin in der Formulierung in einer Konzentration von 300,0 mg pro Dosiseinheit vorliegt.

3. Pharmazeutische Zusammensetzung nach Anspruch 1 und 2, **dadurch gekennzeichnet, dass** Meloxicam in einer Konzentration von 7,5 mg pro Dosiseinheit vorliegt.

4. Pharmazeutische Zusammensetzung nach Anspruch 1 bis 3, **dadurch gekennzeichnet, dass** die genannte Zusammensetzung in einer Einzeldosiseinheit in Kapsel- oder Tablettenform formuliert ist.

5. Pharmazeutische Zusammensetzung nach Anspruch 1 bis 4, **dadurch gekennzeichnet, dass** die genannte Zusammensetzung bei der Behandlung von neuropathischen Schmerzen verwendet wird.

## Revendications

1. Composition pharmaceutique **caractérisée en ce qu'**elle contient de la gabapentine et du méloxicam ainsi que des excipients pharmaceutiquement acceptables : où les plages de concentration pour la gabapentine sont de 3,0 mg à 300,0 mg et de 0,1 mg à 30,0 mg pour le méloxicam : celles-ci sont formulées en une seule unité posologique pour l'administration orale.

2. Composition pharmaceutique selon la revendication 1, **caractérisée en ce que** la gabapentine est présente dans la formulation en une concentration de 300,0 mg par unité de dosage.

3. Composition pharmaceutique selon la revendication 1 ou 2, **caractérisée en ce que** le méloxicam est présent à une concentration de 7,5 mg par unité de dosage.

4. La composition pharmaceutique selon les revendications 1 à 3, **caractérisée en ce que** cette composition est formulée en une seule unité de dosage sous la forme de gélules ou de comprimés.

5. La composition pharmaceutique selon les revendications 1 à 4, **caractérisée en ce que** cette composition est destinée à une utilisation dans le traitement de douleur neuropathique.
